**(19)** Europäisches Patentamt

European Patent Office

Office européen des brevets

**(11)** **EP 1 736 207 A1**

**(12)** **EUROPÄISCHE PATENTANMELDUNG**

**(43)** Veröffentlichungstag:
27.12.2006 Patentblatt 2006/52

**(51)** Int Cl.:
*A61Q 9/04* (2006.01)  *A61K 8/44* (2006.01)
*A61K 8/34* (2006.01)

**(21)** Anmeldenummer: 05013593.8

**(22)** Anmeldetag: 23.06.2005

**(84)** Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

**(71)** Anmelder: **Mibelle AG Cosmetics**
**5033 Buchs (CH)**

**(72)** Erfinder:
• **Marte, Walter**
**9631 SG Ulisbach (CH)**

• **Meyer, Martin**
**8800 Thalwil (CH)**
• **Dutler, Hans**
**8053 Zürich (CH)**
• **Zimmermann, Michael**
**5033 Buchs (CH)**

**(74)** Vertreter: **Forstmeyer, Dietmar et al**
**BOETERS & LIECK**
**Oberanger 32**
**80331 München (DE)**

**(54)** **Zusammensetzung zur Depilierung**

**(57)** Die vorliegende kosmetische Zusammensetzung zur Haarentfernung verwendet Salze der Thioglykolsäure zur Spaltung der Cystin-Schwefel-brücken in Kombination mit einem Entfettungssystem. Die Verwendung eines solchen Systems ermöglicht auch bei einem relativ niedrigen pH-Wert von 10,5 eine schnelle hautschonende Entfernung der Haare.

EP 1 736 207 A1

**Beschreibung**

*Hintergrund der Erfindung*

Die Erfindung betrifft ein chemisches Haarentfernungssystem aus einer Depilierungsformulierung kombiniert mit verschiedenen Applikationssystemen, die eine dem Haarwuchs angepasste minimierte Dosierung der Depilierungsmasse zulassen. Der Anwendungsbereich betrifft sämtliche mit Haar bewachsenen Körperteile inklusiv dem Intim- und Gesichtsbereich.

Die Zielsetzungen derartiger Depilierungssysteme liegen, dem kosmetischen Modetrend folgend, in der raschen, schonenden und schmerzfreien Haarentfernung, ohne Hautirritationen und Geruchsbelästigungen. Sehr wesentlich dabei ist, dass das Haarprotein möglichst rasch fragmentiert und dabei das Hautprotein, insbesondere das Stratum corneum, nicht angegriffen wird. Dies ist durch den unterschiedlichen Aufbau des Haar- und Hautproteins möglich. Das Haarprotein besteht aus $\alpha$-Keratin und Kollagen, wobei die $\alpha$-Helices über Disulfidbrücken miteinander vernetzt sind. Die Festigkeit des Haarproteins wird dadurch verstärkt, dass drei helicale Stränge einander umschlingen und eine Superhelix bilden, deren Zwischenräume Fette unterschiedlicher Zusammensetzung enthalten (z.B. freie Fettsäuren, Triglyceride, freies Cholesterol, Esterwachse) (A.L. Lehninger, D.L. Nelson, M.M. Cox, Prinzipien der Biochemie, Spektrum Akademischer Verlag Heidelberg Berlin Oxford, 1994). Der Anteil an Cystein zur Bildung des Cystins kann bis zu 18 % des Haarproteins betragen. Gemäss diesen Gegebenheiten besteht die Haarfragmentierung in der reduktiven Spaltung des Cystins (Disulfidbrücke) mit der nachfolgenden alkalischen Peptidsolvolyse, die weitgehend diffusionskontrolliert abläuft. Da das Stratum Corneum als wesentliche Schutzschicht der Haut kein Cystin enthält, ist dessen Solvolysemechanismus deutlich unterschiedlich zum Haarprotein. Dies ermöglicht durch die geeignete Wahl der Depilierungsingredienzien eine selektive Fragmentierung des Haarproteins. Durch die Steuerung reaktionstechnischer Parameter des Haarsolvolyseprozesses in den verschiedenen Reaktionsschritten sowie den Einsatz neuer System gerechter Applikationssysteme kann den erwähnten Forderungen Folge geleistet werden.

*Stand der Technik*

Die heute im Handel erhältlichen Depilierungssysteme zeigen in ihrem Design eine grosse Vielfalt. Besonders häufig sind Cremen und Schäume anzutreffen, die sich in ihren chemischen Formulierungen und Applikationsformen nur unwesentlich unterscheiden. Typische Beispiele besagter Anwendungsformen sind: Creme, Spray, Gel, Gel-Creme, kaltes/warmes Wachs, Epilationsgeräte, sowie Nass- und Trockenrasur für Beine, Bikinizonen, Achsel, Gesicht, Intimbereich, und Brusthaare.

Zur Verminderung der Geruchsbelästigung und zum Schutz gegen Bakterienbefall werden den Formulierungen Duftstoffe (z.B. Pfefferminze, Eucalyptus, Nerol, Farnesol, Rosenöl etc.) und Bakterizide (Eucalyptus, p-Hydroxybenzoesäure und deren Ester etc.) beigemischt. Weitere Ingredienzien der Depilierungscremen zur Quellungsverstärkung der Haare sind Harnstoff und dessen Derivate.

Andere Depilierungssysteme, deren Formulierung im Vergleich mit Cremen eine niedrigere Viskosität aufweisen, sind in Verpackungsformen wie Fläschchen, Tiegel, Roll-on, Tuben etc. abgepackt und werden in dünnen Schichten mittels eines Auftragsrollers, Schwammpolsters oder Tuches appliziert. Ein derartiges Beispiel ist Ex-Hair-Roll-on (Firma Medosan). Diese Systeme zeigen aus heutiger Sicht deutliche Vorteile im Vergleich mit Cremen und Schäumen, da die Kontaminationsgefahr durch die Depilierungsmasse mit Körperstellen wie Hände, Augen etc. und die Auftragsdosis der Depilierungsmasse weit geringer ist.

Allen diesen Systemen gemeinsam ist der sehr hohe pH-Wert, der mehrheitlich bei 13 und höher liegt, sowie die relativ hohe Konzentration der depilierend wirkenden Thioglykolsäure, die in den meisten Produkten verwendet wird und deren Konzentration bis zu 0.8 mol/l betragen kann. Die Thioglykolsäure wird in der Regel als Natrium-, Kalium- und/oder Calciumsalz, in Verbindung mit Natron- und Kalilauge sowie Calciumhydroxid eingesetzt. Infolge der nur begrenzt vorhandenen Biokompatibilität handelsüblicher Formulierungen treten Hautirritationen auf, die wiederum durch entsprechende Formulierungsingredienzien wie z.B. Kamillenextrakt (Azulen) und Arnikaextrakt bekämpft werden.

Aufgrund des sehr hohen pH-Wertes der meisten Depilierungsformulierungen sind diese auch nicht im Intimbereich oder an anderen kritischen Körperstellen wie z.B. im Gesicht anwendbar. Weitere standardmässig eingesetzte Ingredienzien sind Verdikkungskomponenten wie z.B. Fettalkohole, Fettsäuren, Sorbitol und Polysaccharide bzw. deren Derivate sowie Dispergatoren und Emulgatoren. Einige wichtige Hersteller solcher Depilierungssysteme sind: Gillette, Reckitt Benkiser, PILCA (GlaxoSmith-Kline), SNEA EPIL (Rufin), X EPIL (Sternmark), NAIR (Church & Dwight), und NADS.

*Aufgabe der Erfindung*

Die Aufgabe der Erfindung besteht darin, eine schnell wirkende, schonende und geruchsarme Zusammensetzung zur schmerzfreien Entfernung von Haaren bereitzustellen, ohne Hautreizungen zu verursachen. Eine weitere Aufgabe besteht darin, ein Applikations-System für die vorstehende Depilierungsformulierung bereitzustellen, das gleichzeitig eine das Haar entfernende Peelingfunktion übernimmt. Bei der Konstruktion des Applikationssystems soll die Gefahr einer unerwünschten Kontamination, z.B. der Hände, durch die Depilierungsmasse minimiert werden und eine umweltgerechte Entsorgung des benutzten Depilierungssystemes möglich sein.

### *Lösung der Aufgabe*

Die Aufgabe wird im Wesentlichen in drei Schritten gelöst.
Der erste besteht im Lösen und gegebenenfalls Emulgieren des Haar schützenden Fettes, mit Hilfe des in der Depilierungsmasse enthaltenen **"Entfettungssystems".** Dadurch wird der Angriff durch die die Solvolyse des Haares auslösenden Wirkstoffe erst ermöglicht.
Der zweite Schritt umfaßt den Solvolyseprozess des Haares, der in Wasserstoffbrücken und Disulfid (Cystin) spaltende sowie untergeordnet in Peptid spaltende Reaktionen zu unterteilen ist. Die entsprechenden Ingredienzien sind im **"Solvolysesystem"** der Depilierungsformulierung zusammengefasst.
Der dritte, optionale Schritt beinhaltet die Entfernung der Depilierungsmasse mit den Haarfragmenten von der Haut durch einen **Peelingprozess** als optionaler Bestandteil des "Depilierungssystems".
Insbesondere betrifft die Erfindung eine kosmetische Zusammensetzung zur Entfernung von Haaren umfassend ein Entfettungssystem mit mindestens einer fettlösenden Verbindung, die eine M-Zahl nach Godfrey von 1 bis 18 besitzt, und ein Solvolyse-System mit mindestens einer Metall-Thioglykolsäure-Verbindung.

### *Beschreibung der Erfindung im Detail*

### 1. Entfernung und Mobilisierung der Fettschicht

Die Haarentfernung erfolgt insbesondere durch Spaltung der Cystin-Schwefelbrücken. Damit die für die Spaltung der Schwefelbrücken erforderlichen Ingredienzien an ihren Wirkungsort gelangen können, ist zunächst die Entfernung der das Haar umgebenden Schutzschicht aus Fett vorteilhaft. Die Entfettung der Haare ist also der erste Schritt und wird durch den Einsatz weitgehend in Wasser unlöslicher, Fett-sorbierender Substanzen in Kombination mit wasserlöslichen Dispergatoren bzw. Emulgatoren und niedermolekularen Fettlösemitteln, vorzugsweise mit Ether-Alkoholen durchgeführt.
Das Entfettungssystem umfaßt drei Bestandteile, nämlich ein Fettlösemittel, ein Fettsorptionsmittel und einen Emulgator. Die einzelnen Bestandteile haben folgende Funktionen: Das Fettlösemittel (Punkt 1.1.) löst das Fett aus dem Haar und mobilisiert, d.h. transportiert es in die öl-Phase der "Öl in Wasser" - Emulsion. Die Öl-Phase dieser Emulsion umfasst mindestens ein Fett-Sorptionsmittel (Punkt 1.2.). Aufgrund des Umstandes, daß das Haarfett durch das Fettlösemittel gelöst und somit beweglich ist, kommt es zu einer Sorption, d.h.
Immobilisierung, des Haarfettes in der Öl-Phase der Emulsion, welche die Fett-Sorptionsmittel umfaßt.
Die Fettlösemittel ermöglichen folglich den Transport des Haarfettes vom Haar in die Öl-Phase der Emulsion, in welcher sie immobilisiert werden. Durch die Immobilisierung der Haarfette in der Öl-Phase der Emulsion wird das Verteilungsgleichgewicht zwischen an Haar sorbierten Fetten und in der wässrigen Lösemittel-Phase gelösten Fetten zugunsten des in der Öl-Phase sorbierten Anteiles verschoben.
Die ferner eingesetzten Dispergatoren bzw. Emulgatoren sind grenzflächenaktive Mittel, welche die Stabilisierung der Öl-Phase in der wässrigen Phase und somit die Bildung der Emulsion bewirken.
**1.1.** Als **Fettlösemittel,** deren Aufgabe es ist, Haar schützende Fette vom Haar zu lösen, zu mobilisieren, d.h. den Transport in die Öl-Phase der Emulsion zu ermöglichen, werden polare, protische Lösemittel eingesetzt. Bevorzugte Beispiele hierfür sind Alkohole mit 1, 2, 3, 4 oder 5 Kohlenstoffatomen, und deren Ether.
Die eingesetzten Fettlöser, deren M-Zahlen im Bereich von 1 - 18 liegen, zeichnen sich sowohl durch ihre gute Wasser- als auch Fettlöslichkeit aus. Die M-Zahlencharakterisierung von Lösemitteln basiert gemäss den Arbeiten von Godfrey auf deren Affinität für "Öl-ähnliche Substanzen" (N.B. Godfrey, Chem-Tech, June 1972, S. 359).
Allgemein sind Fettlösemittel im Sinne dieser Erfindung polare, protische, wasser-lösliche, flüssige Verbindungen.
Typische Vertreter sind Alkohole mit 1, 2, 3, 4, oder 5 Kohlenstoffatomen, und Ether-Alkohole, niedermolekulare, wasserlösliche Ethylenoxid-Homopolymere (zum Beispiel Pluriol E mit einem Molekulargewicht von 102 bis 2.000 der Fa. BASF) und Propylenoxid-Homopolymere (zum Beispiel Pluriol P mit einem Molekulargewicht von 134 bis 600 der Fa. BASF) und deren Derivate, niedermolekulare, statistische und wasserlösliche Ethylenoxid- und Propylenoxid-Copolymere und deren Derivate, niedermolekulare, wasserlösliche Ethylenoxid- und Propylenoxid-Blockcopolymerisate (zum Beispiel Pluronic PE mit einem Molekulargewicht von 1.000 bis 4.000 und Pluronic RPE mit einem Molekulargewicht von 2.000 bis 4.000) und deren Derivate, sowie Ether-Alkohole wie zum Beispiel Ethoxyethanol (Ethoxyglykol), Propoxyethanol (Propoxyglykol), Butoxyethanol (Butoxyglykol), Butylacetat und Glycerintriacetat. Als sehr geeignet haben sich Pluronic, insbesondere Pluronic PE 4300 (Firma BASF), und Butoxyglykol erwiesen.

Die Einsatzmenge dieser polaren, protischen Lösemittel liegen im Bereich von 1 - 15 Gew.-%, vorzugsweise 3 - 6 Gew.-%, bezogen auf gesamte kosmetische Zusammensetzung.

**1.2.** Die durch die Lösemittel mobilisierten Fette werden vorzugsweise entsprechend ihrer Löslichkeit in der wässrigen Lösemittel-Phase von den optional einsetzbaren, **Fett sorbierenden Verbindungen** des "Entfettungssystems" immobilisiert, wodurch weiteres Fett in der wässrigen Phase gelöst werden kann. Allgemein sind Fett-sorbierende Verbindungen im Sinne dieser Erfindung weitgehend wasser-unlöslich, d.h. weniger als 1 %; daher werden sie als Öl-Phase in Wasser emulgiert. Die Fett sorbienden Verbindungen stellen im wesentlichen die Öl-Phase der "Öl in Wasser" - Emulsion dar.

Als **Fett-sorbierende** und - vorzugsweise gleichzeitig die Depilierungsmasse verdickende - Komponenten werden - neben Mineralölen - Fettalkohole, Fettsäuren und Fettamide mit jeweils 12, 13, 14, 15, 16, 17, 18, 19, 20, 21, 22, 23, oder 24 Kohlenstoffatomen eingesetzt. Diese Verbindungen haben die Aufgabe, das bereits durch die Fettlösemittel gelöste Fett in der wässrigen Phase der "Öl in Wasser"-Emulsion zu immobilisieren, damit durch die Fettlöser neues Fett gelöst werden kann.

Typische Vertreter dieser Substanzklassen sind Cetylalkohol, Stearinsäure und deren Veresterungsderivate wie z.B. Octyl- und Cetylpalmitate und Triglyceride, sowie ethoxylierte und propoxylierte Fettalkohole mit jeweils 12, 13, 14, 15, 16, 17, 18, 19, 20, 21, 22, 23, oder 24 Kohlenstoffatomen im Fettalkohol; die Zahl der Ethylenoxid-bzw. Propylenoxid-Einheiten in den ethoxylierten bzw. propoxylierten Fettalkoholen beträgt 1 bis 10, vorzugsweise 1 - 5.

Neben den bereits erwähnten Substanzen sind Silizium organische Verbindungen wie z,B, Phenyldimethicone, Cetyldimethicone oder Cyclomethicone ebenfalls sehr gut geeignet. Vorzugsweise eingesetzte Produkte sind Cetyl- und Cetearylalkohol.

Die Fett-sorbierenden Substanzen werden im Konzentrationsbereich von 2 - 15 Gew.-% eingesetzt, vorzugsweise von 5 - 10 Gew.-%, bezogen auf die gesamte kosmetische Zusammensetzung.

**1.3. Als Dispergatoren bzw. Emulgatoren, die Bestandteile** des Entfettungssystems sein können, können grenzflächenaktive Substanzen mit HLB-Werten zwischen 7 und 18 eingesetzt werden (Hydrophilic-Lipophilic-Balance, siehe H. Sonntag, Lehrbuch der Kolloidwissenschaft, VEB Deutscher Verlag der Wissenschaften Berlin 1977). Als besonders geeignete Vertreter dieser Substanzklasse sind Laurylsulfat, Laurethsulfat, Span 20 (Sorbitanlaurat), Tween 20 (ethoxyliertes Sorbitanlaurat), Brij 58 (Polyoxyethylen-(20)-cetylether), Renex 720 (ethoxylierter $C_{13}$ - $C_{15}$ - Alkohol) und Laureth-23. Speziell geeignet ist eine Kombination bestehend aus $^2/_3$ Laurethsulfat und $^1/_3$ Laurylethoxylat.

Die Gesamteinsatzmenge Grenzflächen aktiver Substanzen liegt im Bereich von 2 - 10 Gewichtsprozenten, vorzugsweise 4 - 6 % bezogen auf die gesamte kosmetische zusammensetzung.

**1.4. Verdickungsmittel für die wässrige Phase**

Darüber hinaus können Verdickungsmittel für die wässrige Phase der "Öl in Wasser"-Emulsion eingesetzt werden. Damit kann die Viskosität der kosmetischen Zusammensetzung gesteigert und ihre Handhabung erleichtert werden.

Als typische Vertreter für Verdickungsmittel der wässrigen Phase kommen in Betracht: Natrium-Alginate, insbesondere Alginate EHV (CHT-Tübingen), Polyacrylate, zum Beispiel Dynasorb der Fa. Stockhausen, sowie Cellulose-Derivate, zum Beispiel Methocel 311 der Fa. Dow Chemical Company oder Bermocell E 230-FQ der Fa. Akzo Nobel.

**2. Solvolysesystem.**

**2.1. Lösemittel-lösliche Metallkomplexe der Thioglykolsäure**

Ein Gesichtspunkt dieser Erfindung betrifft die Verwendung von Metallkomplexen der Thioglykolsäure, die teilweise in polaren, protischen Lösemitteln, zum Beispiel Ethanol, teilweise löslich sind. Insbesondere sind diese Metallkomplexe in den unter Punkt 1.1. aufgeführten Fettlösemitteln teilweise löslich.

Die Solvolyse des Haares, als zweiter Schritt, wird vornehmlich durch die Tertiär- und Sekundärstruktur spaltenden Depilierungsingredienzien bewerkstelligt ("Solvolysesystem").

Als Cystin spaltende Ingredienzien können neben den bekannten Natrium-, Kalium- oder Calcium-Thioglykolaten erfindungsgemäss zumindest teilweise in Alkohol lösliche Metall-Thioglykolsäure-Komplexe der A- und B-Kationen, wie Aluminium- oder Zink-Thioglykolsäure-Salze (siehe hierzu: A. Ackermann, W_ Jugelt, H.H. Möbius, H.D. Suschke, G. Werner, Elektrolytgleichgewichte und Elektrochemie, Verlag Chemie Weinheim, 1974, Seiten 221 - 223) verwendet werden.

Damit die Spaltungsreagenzien eine Solvolyse des Haares durchführen können ist, wie vorstehend angegeben, zunächst eine Entfernung der das Haar umgebenden Schutzschicht aus Fett erforderlich. Diese Entfernung erfolgt üblicherweise durch das vorstehend erwähnte Entfettungssystem.

Die vorliegenden Erfinder haben in unerwarteter Weise gefunden, daß Aluminium- bzw. Zink-Komplexe der Thioglykolsäure die Spaltung der Cystin-Schwefelbrücken beschleunigen. Während die herkömmlicherweise eingesetzten Natrium-, Kalium- und Calciumsalze der Thioglykolsäure in erster Linie wasserlöslich, nicht jedoch in den Lösemitteln löslich sind, zeichnen sich die in dieser Erfindung eingesetzten Aluminium- und Zink-Komplexe der Thioglykolsäure durch eine deutliche Löslichkeit in den Lösemitteln, z.B. in den Alkoholen aus, die auch als Fettlösemittel (siehe Punkt 1.1.) eingesetzt werden können.

Die Thioglykolsäure des Aluminiums und des Zinks grenzen sich von den üblicherweise verwendeten Natrium-, Kalium- und Calciumsalzen dadurch ab, dass letztere praktisch vollständig dissoziiert sind, d.h. als Salze vorliegen und daher in den unter Punkt 1.1. aufgeführten Fettlösemitteln nicht löslich sind. Demgegenüber liegen die Aluminium- bzw. zink-verbindungen der Thioglykolsäure nicht als Salze vor, sondern sind undissoziierte Komplexe, die eine deutliche Löslichkeit in den Fettlösemitteln im Sinne dieser Erfindung (Punkt 1.1.), beispielsweise Ethanol, aufweisen.

Dadurch wird bereits eine Spaltung der Cystin-Schwefelbrücken des Haares eingeleitet, während der Entfettungsprozess noch andauert. Auf diese Weise wird eine besonders schnelle Haarentfernung ermöglicht, indem der Entfettungsprozess und der Solvolyseprozess zumindest teilweise gleichzeitig ablaufen.

Vorzugsweise eingesetzte Thioglykolsäurekomplexe beinhalten Aluminium und Titan als Beispiele für das A-Kation bzw. Zink und Zinn für das B-Kation. Die Komplexe der Thioglykolsäure werden in einer Konzentration von 0,2 M bis 0,8 M, bevorzugt von 0,2 M bis 0,4 M, insbesondere bevorzugt etwa 0,3 M eingesetzt.

Gegebenenfalls können zusätzlich Alkali-Salze der Thioglykolsäure eingesetzt werden.

Ferner kann die kosmetische Zusammensetzung mindestens ein reduzierendes Endiol oder Endiolat oder deren Derivate umfassen. Beispiele derartiger Endiole oder Endiolate sind Ascorbinsäure, Ketozucker wie Fruchtzucker, sowie Acetoin und Adepoin. Als stark reduktiv wirkende Endiolate auch Reduktone genannt sind Ketozucker (z.B. Fructose), Acetoin und Adepoin hervorzuheben. Die Endiol-Verbindungen können in einer Konzentration von 0,2 M bis 0,5 M, bevorzugt 0,2 M bis 0,3 M eingesetzt werden.

Eine weitere und sehr effiziente Möglichkeit, die Cystinspaltungsgeschwindigkeit zu erhöhen, ist durch den Einsatz von Dithiothreitol gegeben, da das Reaktionsgleichgewicht der Disufidspaltung zu Gunsten des Dithiothreitol-Produktes verschoben wird. Dithiothreitol kann in einer Konzentration von 0,2 M bis 0,8 M, bevorzugt von 0,2 M bis 0,4 M eingesetzt werden.

## 2.2. Tertiäre Amine als Katalysatoren: DABCO und DBU

Zusätzlich kann das Solvolyse-System mindestens ein tertiäres Amin umfassen; durch derartige Amine wird die Solvolyse von Haar und insbesondere die Spaltung von Wasserstoffbrücken und untergeordnet auch von Peptiden katalysiert.

Allgemein sind tertiäre Amine im Sinne dieser Erfindung cyclische Amine mit mindestens einer, vorzugsweise zwei tertiären Amin-Funktionen und mindestens einem Ring, die nicht unter Bedingungen der Depilierung hydrolysieren, d.h. pH kleiner oder gleich 11,5; Temperatur kleiner oder gleich 37°C. Insbesondere sind solche Amine bevorzugt, die der Struktur $NR^1R^2R^3N$ entsprechen, wobei $R^1$, $R^2$, und $R^3$ jeweils unabhängig geradkettige oder verzweigte, gesättigte oder ungesättigte, substituierte oder unsubstituierte Kohlenwasserstoff-Gruppen mit 2, 3, 4, 5, 6 oder 7 Kohlenstoffatomen darstellen, wobei jeweils zwei dieser Gruppen zusammen einen Ring bilden können.

Als sehr effizient haben sich hierbei die Substanzen 1,4-Diazabicyclo [2,2,2] octan (DABCO), 1,8-Diazabicyclo [5.4.0]undec-7-en (DBU), 1,4-Diazabicyclo[4.3.0]non-5en und Triethanolamin $N(CH_2CH_2OH)_3$ erwiesen.

## 2.3. Schonung der Haut durch niedrigen pH-Wert: Puffersystem

Durch die Verwendung der tertiären, nicht reaktiven Amine als Solvolysekatalysatoren ist es möglich, die Depilierung bei pH-Werten von 10 - 11.5 vorzugsweise bei pH 10.5 - 11, anstelle des sonst üblichen pH-Wertes von 13 und höher, durchzuführen. Dadurch kann die Entfernung der Haare wesentlich hautschonender durchgeführt werden; trotz des erniedrigten pH-Wertes im Vergleich zu herkömmlichen Depilierungsmassen wird durch die Katalysatoren eine ausreichende Geschwindigkeit für die Solvolyse der Peptid-Bindungen erzielt.

Die Einstellung des pH-Wertes der kosmetischen Zusammensetzung erfolgt durch den Einsatz von Puffersystemen wie z.B. Kaliumhydrogencarbonat / Kaliumcarbonat für einen pH-Bereich von 9,5 bis 11,0, dessen Puffer-Maximum bei etwa 10.5 liegt, sowie Siliziumdioxid / Natronlauge (Wasserglas) für einen pH-Bereich von 11 - 12, dessen Puffer-Maximum bei etwa 11 liegt. Die Einsatzmenge der korrespondierenden Säure-Basenpaare ist so zu wählen, dass in der kosmetischen Zusammensetzung dieser Erfindung eine Pufferkapazität von 0.02 - 0.06 mol/l, vorzugsweise 0.04 - 0.05 mol/l vorherrscht.

Durch die Verwendung tertiärer Amine wie DABCO und DBU sowie durch den Einsatz bei pH 10,5 bis 11,5 bewegen sich die Depilierungszeiten je nach Haarstärke und Fettungsgrad der Haare zwischen 3 und 8 Minuten, üblicherweise zwischen 4 und 6 Minuten.

Darüber hinaus kann durch die Verwendung des erfindungsgemäßen Katalysatorsystems die Menge der einzusetzenden Thioglykolsäure bzw. von deren Salzen bzw. Derivate reduziert werden, so dass durch die im Vergleich zu herkömmlichen Depilierungsmitteln geringe Konzentration an Thioglykolsäure eine merkliche Verminderung der Geruchsbelästigung erreicht werden kann.

### 3. Beispiele und experimenteller Teil

Die Basisinformationen zur Entwicklung der erfindungsgemässen Depilierungsformulierung wurden sowohl aus kinetischen Daten der Haarsolvolyse als auch aus den Haarbruchdaten gewonnen.
Als Prüflinge dienten Methanol gereinigte, ca. 300 mm lange Frauenhaare.

Zur Messung der Solvolysekinetik wurde das Haar in die entsprechende Depilierungsflotte getaucht, mit einem Vorspanngewicht von 300 - 350 mN belastet und die in Folge der Haarfragmentierung abnehmende Vorspannkraft in der zeitlichen Abfolge gemessen. Zur Erfassung der Bruchzeit wurde das Haar mit einer konstanten Vorspannkraft von 295 mN belastet und bis zum Bruch in die entsprechende Depilierungsflotte getaucht. Das der Arbeitshypothese zu Grunde gelegte Reaktionsschema (Abb. 1) beschreibt eine Folgereaktion pseudo erster Ordnung, mit einem in der ersten Reaktionsstufe, d.h. zwischen A und B mit den Geschwindigkeitskonstanten $k_1$ und $k_{-1}$, auftretenden Gleichgewichtsschritt, der hauptsächlich der Disulfidspaltung zuzuordnen ist. Der zweite, irreversible Reaktionsschritt von B nach C mit der Geschwindigkeitskonstante $k_2$ entspricht der diffusionskontrollierten Wasserstoffbrücken- und der ebenfalls möglichen Peptidspaltung.

$$A \underset{k_{-1}}{\overset{k_1}{\rightleftharpoons}} B \xrightarrow{k_2} C$$

Abb.1  Reaktionschema der Haarsolvolyse (Cystin- und Wasserstoffbrücken spaltende, die Sekundär- und Tertiärstrukturen des Haares auflösenden Reaktionsstufen)

Als Zielgrössen zur Beschreibung der Wirkungsweisen der Depilierungsingredienzien werden die Reaktionskonstanten des ersten und zweiten Reaktionsschrittes (Abb. 1) sowie die Haarbruchzeit ermittelt und normiert. Die Bestwerte (grösste Reaktionskonstante und geringste Bruchzeit) entsprechen in der normierten Skala der Abbildung 2 Eins und die schlechtesten Werte Null. Durch die Zusammenführung der normierten zielfunktionswerte der drei Zielgrössen zu unimodalen Funktionswerten $Z_i$ und die anschliessende semiempirische Modellierung wird eine die Wirkungsweisen der Depilierungsingredienzien beschreibende Funktion erhalten.

Eine weitere wesentliche Information ist aus der Abbildung 2 zu ersehen. Sie zeigt die Isolinien der normierten Zielgrösse (Depilierungsgeschwindigkeit) in Abhängigkeit des pH-Wertes und der Methanolkonzentration bei konstanter Thioglykolsäure- und DABCO-Konzentration (Abb. 2).

Legende:

Z = "norm. Solvolysegeschw. [—]"

x-Achse:   x2 = "pH-Wert [--]"
y-Achse:   x1 = "Methanol [mol/l]"

Konst. Einstellungen

x0 = "Thiogs [mol/l]"    $x_0 = 0.3$
x3 = "DABCO [mol/l]"    $x_3 = 0.025$

Z1

Abb. 2.   Isolinien der normierten Depilierungsgeschwindigkeit in Abhängigkeit des pH-Wertes und der Methanolkonzentration:

Abszisse: pH-Wert;
Ordinate: Methanolkonzentration in mol/l;
Konstante Werte; 0,3 M Kalium-Thioglykolat und Konzentration des tertiären Amins 0,025 M DABCO.

Die zur Depilierung günstigsten Reaktionsbedingungen liegen gemäss Abbildung 2 bei ca. pH 10 und 3 mol/l Methanol.

Eine weitere wichtige Information ist der Abbildung 3 zu entnehmen. Sie zeigt die Isolinien der normierten Zielgrösse in Abhängigkeit des pH-Wertes und der DABCO-Konzentration mit den bestmöglichen Reaktionsbedingungen bei pH 10 - 10.5 und 0.02 - 0.03 mol/l DABCO. Die Konzentration des Kalium-Thioglykolats beträgt konstant 0.3 mol/l und die Methanol-Konzentration konstant 2 mol/l. Man erkennt, daß eine Konzentration von DABCO von 0,025 mol/l und ein pH-Wert von ca. 10,2 zu optimalen Depilierungs-Geschwindigkeit führt. Dennoch wird die Depilierung bei einem pH-Wert von etwa 10,5 durchgeführt, der auch der maximalen Pufferkapazität des Systems Kaliumhydrogencarbonat / Kaliumcarbonat entspricht, um stets die Funktionstüchtigkeit zu gewährleisten.

Abb. 3   Isolinien der normierten Depilierungs-Geschwindigkeit in Abhängigkeit des pH-Wertes und der DABCO-Konzentration
Abszisse: pH-Wert
Ordinate: Konzentration von DABCO in mol/l
Konstante Werte: 0,3 M Kalium-Thioglykolat
und 2 M Methanol

Zur Messung der Solvolysekinetik wurde das Haar in die entsprechende Depilierungsflotte getaucht, mit einem Vorspanngewicht von 300 - 350 mN belastet und die in Folge der Haarfragmentierung abnehmende Vorspannkraft in der zeitlichen Abfolge gemessen. Die Erfassung der Haarbruchzeit erfolgte bei konstanter Vorspannkraft von 295 mN und variablen Konzentrationen der Depilierungsingredienzien.

## 4. Handschuh als Peelingsystem

Zur Applikation der Depilierungsmasse dient erfindungsgemäss ein "Handschuh", dessen eine Seite mit der Depilierungsmasse beschichtet ist und die Rückseite aufgrund der rauen Oberfläche als Peelinghandschuh zur Entfernung der Depilierungsmasse und der Haare verwendet wird. Die Fertigung eines derartigen Handschuhs ist äusserst kostengünstig, da die Handschuhe aus einem mehrlagigen Paket konfektioniert werden können. Die oberste Schicht des dreilagigen Paketes besteht aus einem mit der Depilierungsmasse beschichteten Faservlies, die Mittelschicht ist beispielsweise eine Polyethylenfolie als Kontaminierungsbarriere und die letzte entspricht der Peelingschicht die aus einem rauhen Gewebe, Gewirk oder Vlies gefertigt ist. Zur Handschuhkonfektionierung wird die beschichtete, dreilagige, bis zu 2500 mm breite Warenbahn in ca. 150 mm breite Einzelbahnen geschnitten und an den Rändern vernäht bzw. verschweisst. Anschliessend werden die Einzelbahnen in 150 mm lange Einzelstücke geschnitten und nurmehr einseitig vernäht oder verschweisst, so dass ein Handschuh entsteht, dessen eine Seite die Depilierungsmasse trägt und die andere zur Reinigung (peeling) verwendet werden kann. In anderen Fällen ist es sinnvoll, die Vernähung bzw. Verschweissung des dreilagigen Applikationssysems bereits vor der Beschichtung durchzuführen.

Eine zweite Möglichkeit besteht in der Verwendung einer mit Klebstoff beschichteten Kunststofffolie in deren Beschichtungsmasse die zuvor erwähnten Depilierungschemikalien eingearbeitet sind, Die beschichtete Seite der Folie wird mit einer zweiten Folie unmittelbar nach dem Beschichtungsprozess abgedeckt und gelangt als Warenbahn in die Konfektionierung. Die Haarentfernung mit der beschichteten Klebfolie erfolgt durch die Entfernung der Schutzfolie und Anpressung der beschichteten Folienseite an die entsprechende, zu enthaarende Körperstelle. Nach einer Einwirkzeit von 3 - 8 Minuten wird die Folie abgezogen und mit ihr die in Auflösung befindlichen Haare. Diese Art der Haarentfernung stellt eine Kombination aus Depilierung und Epilierung dar.

Beide erwähnten Applikationsformen zeichnen sich durch ihre einfache, und kostengünstige Herstellung aus und sind ausserdem umweltfreundlich zu entsorgen.

## Beispiel 1 Depilierungsformulierung und Applikationssystem

Die in der nachfolgend aufgeführten Tabelle aufgelisteten **Entfettungs**chemikalien werden bis zur Schmelze erhitzt und vermischt und anschliessend in der Hälfte des zur Verfügung stehenden Wassers emulgiert. Die im Solvolysesystem aufgeführten Chemikalien werden in einer Wasservorlage gelöst, um anschliessend die zwischenzeitlich abgekühlte "Entfettungsemulsion" in der "Solvolyselösung" zu emulgieren. Nach erfolgter Emulgierung wird das restliche Wasser bis zu einem Volumen von einem Liter zugegeben.

Nun wird die Depilierungsformulierung mittels eines Beschichtungsprozesses auf die Vliesseite (Vliesstärke 1.5 mm) einer dreischichtigen Warenbahn (Vlies/Polyethylenfolie/Peelinggewirk) appliziert, die dann mit einer Schutzfolie überzogen wird. Die Beschichtungsauflage beträgt 500 g/m$^2$. Mit dieser Beschichtungsauflage kann pro "Handschuh" die Fläche eines Beines depiliert werden.

## Beispiel: Rezeptur der Depilierungsmasse

| Funktionssystem | Ingredienzien | Funktion | Konzentration |
|---|---|---|---|
| **Entfettungs-System** | Cetylalkohol | Verdickung und Fettsorbierung | 70.0 g/l |
| | Laurylsulfat | Emulgator anionisch | 15.0 g/l |
| | Inbetin 180 (Kolb AG) | Emulgator nichtionogen | 15.0 g/l |
| | Pluronic PE 4300 (BASF) | Fettsolubilisierung | 5.0 g/l |
| | 2-Butoxyglykol | Fettlöser | 10.0 g/l |
| | Ethanol | Fettlöser | 50.0 g/l |
| **Solvolyse-System** | Kalium-Thioglykolat | Cystinspaltung | 0.4 mol/l |
| | DABCO | Katalysator der Haarsolvolyse | 0.025 mol/l |
| | $SiO_2$/NaOH (Wasserglas) | pH-Wert Einstellung auf 11 | 0.5 mol/l |
| | Alginat EHV (CHT-Tübingen) | Verdicker der Wasser-Phase | 3.0 g/l |

Das Peelinggewirk, als Reinigungsschicht des Handschuhs, ist aus groben Polyethylenfasern gefertigt, um so die stark fragmentierten Haare, inklusiv der Depilierungsmasse zu entfernen. Die auf diese Weise hergestellte Depilierungsformulierung zeichnet sich durch ihre geringe Geruchsbelästigung, den niedrigen pH-Wert sowie durch die kurze Depilierungszeit von 4 Minuten aus. Aufgrund des niedrigen pH-Wertes wurden auch bei Unterlassung einer Depilierungsnachbehandlung keine Hautirritationen festgestellt.

## Patentansprüche

1. Kosmetische Zusammensetzung zur Entfernung von Haaren umfassend

   - ein Entfettungssystem mit mindestens einer wasserlöslichen, fettlösenden Verbindung, die eine M-Zahl nach Godfrey von 1 bis 18 besitzt, und
- ein Solvolyse-System mit mindestens einer Metall-Thioglykolsäure-Verbindung.

2. Kosmetische Zusammensetzung nach Anspruch 1, wobei die fettlösende Verbindung ausgewählt ist aus:

- Alkoholen mit 1, 2, 3, 4, oder 5 Kohlenstoffatomen, und
- niedermolekularen, wasserlöslichen Ethylenoxid-Homopolymeren und Propylenoxid-Homopolymeren und deren Derivaten,
- niedermolekularen, wasserlöslichen, statistischen Ethylenoxid- und Propylenoxid-Copolymeren und deren Derivaten,
- niedermolekularen wasserlöslichen Ethylenoxid- und Propylenoxid-Blockcopolymerisaten und deren Derivaten,
- Ether-Alkoholen wie Ethoxyethanol (Ethoxyglykol), Propoxyethanol (Propoxyglykol), Butoxyethanol (Butoxyglykol), oder
- sowie Butylacetat und Glycerintriacetat.

3. Kosmetische Zusammensetzung nach Anspruch 1 oder 2, wobei die fettlösenden Verbindungen aus niedermolekularen wasserlöslichen Ethylenoxid- und/oder Propylenoxid-Blockcopolymerisaten, bevorzugt Pluriol E, Pluriol P, Pluronic PE, Pluronic RPE, alle von der Fa. BASF, insbesondere bevorzugt Pluronic PE 4300 (Fa. BASF) ausgewählt sind.

4. Kosmetische Zusammensetzung nach einem der vorstehenden Ansprüche, wobei die Zusammensetzung mindestens eine das Fett sorbierende Verbindung umfaßt.

5. Kosmetische Zusammensetzung nach Anspruch 4, wobei die das Fett sorbierende, wasserunlösliche Verbindung ausgewählt ist aus:

- einem Mineralöl,
- Fettalkoholen, Fettsäuren und Fettamiden mit jeweils 12, 13, 14, 15, 16, 17, 18, 19, 20, 21, 22, 23, oder 24 Kohlenstoffatomen, wie Cetylalkohol, Stearinsäure, und deren Veresterungsprodukten,
- Triglyceriden
- ethoxylierten und propoxylierten Fettalkoholen mit 12, 13, 14, 15, 16, 17, 18, 19, 20, 21, 22, 23, oder 24 Kohlenstoffatomen im Fettalkohol sowie 1 bis 10, vorzugsweise 1 bis 5 Ethylenoxid- bzw. Propylenoxid-Einheiten, oder
- siliciumorganischen Verbindungen.

6. Kosmetische Zusammensetzung nach Anspruch 5, wobei die das Fett sorbierende Verbindung aus Octylpalmitat, Cetylpalmitat, Phenyldimethicone, Cetyldimethicone, Cyclomethicone ausgewählt ist.

7. Kosmetische Zusammensetzung nach einem der vorstehenden Ansprüche, wobei das Solvolyse-System mindestens eine Natrium-, Kalium-, Calcium-, Aluminium-, Titan-,.Zinn- oder Zink-Thioglykolsäure-Verbindung, und/oder Dithiothreitol umfaßt, bevorzugt eine Aluminium- und/oder Zink-Thioglykolsäure-Verbindung.

8. Kosmetische Zusammensetzung nach einem der vorstehenden Ansprüche, wobei die Zusammensetzung mindestens ein reduzierendes Endiol oder dessen Derivat umfaßt, insbesondere Ascorbinsäure, oder Ketozucker wie Fructose, sowie Acetoin und Adepoin.

9. Kosmetische Zusammensetzung nach einem der vorstehenden Ansprüche, wobei das Solvolyse-System mindestens ein tertiäres Amin umfaßt, insbesondere 1,4-Diazabicyclo[2.2.2]-octan (DABCO), 1,8-Diazabicyclo[5.4.0]undece-7-en (DBU), 1,4-Diazabicyclo[4.3.0]non-5en und/oder Triethanolamin umfaßt.

10. Kosmetische Zusammensetzung nach einem der vorstehenden Ansprüche, wobei die Zusammensetzung ein Puffer-System umfaßt, und einen pH-Wert von 10,0 bis 12,0, vorzugsweise von 10,5 bis 11,0 besitzt.

11. Kosmetische Zusammensetzung nach Anspruch 10, wobei das Puffer-System

- Kaliumhydrogencarbonat / Kaliumcarbonat für einen pH-Wert von etwa 10,5, und/oder
- Siliciumdioxid / Natronlauge für einen pH-Wert von etwa 11 umfaßt.

12. Kosmetische Zusammensetzung nach einem der vorstehenden Ansprüche, wobei die Zusammensetzung ein grenzflächenaktives Mittel mit einem HLB-Wert von 7 bis 18 umfaßt.

13. Kosmetische Zusammensetzung nach Anspruch 12, wobei das grenzflächenaktive Mittel Laurylsulfat, Laurethsulfat, Span 20, Tween 20, Brij 58, Renex 720, Laureth-23, Inbetin 180, insbesondere bevorzugt eine Mischung aus 2/3 Laurethsulfat und 1/3 Laurylethoxylat ist.

14. Kosmetische Zusammensetzung nach einem der vorstehenden Ansprüche, wobei die Zusammensetzung ein Verdickungsmittel für die wässrige Phase der Emulsion umfaßt.

15. Kosmetische Zusammensetzung nach Anspruch 14, wobei das Verdickungsmittel Cetylalkohol, Natrium-Alginate, Polyacrylate und/oder Cellulose-Derivate umfaßt.

16. Verwendung der kosmetischen Zusammensetzung nach einem vorgehenden Ansprüche zur Entfernung von Haaren.

17. Folienanordnung zur Entfernung von Haaren, mit einer Trägerfolie, wobei die Trägefolie

   a) auf einer Seite mit der kosmetischen Zusammensetzung nach einem der vorstehenden Ansprüche beschichtet ist.

18. Folienanordnung nach Anspruch 17, wobei die Schicht a) zusätzlich einen Klebstoff aufweist.

19. Folienanordnung nach Anspruch 17 oder Anspruch 18, wobei auf der Schicht a) eine abziehbare Schutzfolie b) angeordnet ist.

20. Folienanordnung in Form eines Handschuhs, dessen eine Oberfläche eine Trägerfolie mit einer kosmetischen Zusammensetzung nach einem der Ansprüche 1 bis 15 aufweist und dessen andere Oberfläche mit einer rauhen Oberfläche versehen ist.

**Europäisches Patentamt**

## EUROPÄISCHER RECHERCHENBERICHT

Nummer der Anmeldung

EP 05 01 3593

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 589 516 B1 (EYRE HEATHER ET AL) 8. Juli 2003 (2003-07-08) * Spalte 10, Zeilen 7-9; Beispiel 7 * ----- | 1-20 | A61Q9/04 A61K8/44 A61K8/34 |
| X | GB 2 367 749 A (* RECKITT BENCKISER FRANCE) 17. April 2002 (2002-04-17) * Seite 1, Zeilen 5-12; Beispiele 1-4 * * Seite 1, Zeilen 20,21 * ----- | 1-20 | |
| X | WO 99/20241 A (RHODIA CHIMIE) 29. April 1999 (1999-04-29) * Beispiel 4 * ----- | 1-20 | |
| X | US 2004/219118 A1 (SLAVTCHEFF CRAIG STEPHEN ET AL) 4. November 2004 (2004-11-04) * Absatz [0023]; Tabelle III * ----- | 1-20 | |
| X | EP 1 402 879 A (JOHNSON & JOHNSON CONSUMER [US]) 31. März 2004 (2004-03-31) * Beispiel 9 * ----- | 1-20 | |
| X | US 2002/146380 A1 (NAMBU HIROMI ET AL) 10. Oktober 2002 (2002-10-10) * Beispiele 1-7 * ----- | 1-20 | RECHERCHIERTE SACHGEBIETE (IPC) A61K |
| X | US 2003/165429 A1 (TAKEOKA ERIKO ET AL) 4. September 2003 (2003-09-04) * Beispiel 6 * ----- | 1-20 | |

Der vorliegende Recherchenbericht wurde für alle Patentansprüche erstellt

| Recherchenort | Abschlußdatum der Recherche | Prüfer |
|---|---|---|
| München | 28. November 2005 | Miller, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
 
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 05 01 3593

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-11-2005

| Im Recherchenbericht angeführtes Patentdokument | | Datum der Veröffentlichung | Mitglied(er) der Patentfamilie | | | Datum der Veröffentlichung |
|---|---|---|---|---|---|---|
| US 6589516 | B1 | 08-07-2003 | AT | 243524 | T | 15-07-2003 |
| | | | AU | 3443200 | A | 16-10-2000 |
| | | | BR | 0009340 | A | 23-04-2002 |
| | | | DE | 60003537 | D1 | 31-07-2003 |
| | | | DE | 60003537 | T2 | 24-12-2003 |
| | | | EP | 1165107 | A1 | 02-01-2002 |
| | | | WO | 0057893 | A1 | 05-10-2000 |
| GB 2367749 | A | 17-04-2002 | KEINE | | | |
| WO 9920241 | A | 29-04-1999 | AU | 746945 | B2 | 09-05-2002 |
| | | | AU | 9269998 | A | 10-05-1999 |
| | | | BR | 9813118 | A | 22-08-2000 |
| | | | CA | 2307321 | A1 | 29-04-1999 |
| | | | CN | 1280486 | A | 17-01-2001 |
| | | | EP | 1024782 | A1 | 09-08-2000 |
| | | | FR | 2769836 | A1 | 23-04-1999 |
| | | | JP | 2001520180 | T | 30-10-2001 |
| US 2004219118 | A1 | 04-11-2004 | KEINE | | | |
| EP 1402879 | A | 31-03-2004 | BR | 0304289 | A | 31-08-2004 |
| | | | CA | 2443329 | A1 | 30-03-2004 |
| | | | US | 2004062735 | A1 | 01-04-2004 |
| US 2002146380 | A1 | 10-10-2002 | CA | 2369276 | A1 | 26-07-2002 |
| | | | JP | 2002293723 | A | 09-10-2002 |
| US 2003165429 | A1 | 04-09-2003 | KEINE | | | |

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **A.L. LEHNINGER ; D.L. NELSON ; M.M. COX.** Prinzipien der Biochemie. Spektrum Akademischer Verlag, 1994
- **N.B. GODFREY.** Öl-ähnliche Substanzen. *Chem-Tech,* Juni 1972, 359

- **H. SONNTAG.** *Lehrbuch der Kolloidwissenschaft,* 1977
- **A. ACKERMANN ; W_ JUGELT ; H.H. MÖBIUS ; H.D. SUSCHKE ; G. WERNER.** *Elektrolytgleichgewichte und Elektrochemie,* 1974, 221-223